(19) Europäisches Patentamt / European Patent Office / Office européen des brevets

(11) **EP 1 468 151 B1**

(12) **EUROPÄISCHE PATENTSCHRIFT**

(45) Veröffentlichungstag und Bekanntmachung des Hinweises auf die Patenterteilung:
**07.05.2008 Patentblatt 2008/19**

(21) Anmeldenummer: **03701510.4**

(22) Anmeldetag: **14.01.2003**

(51) Int Cl.:
*E04H 4/12* *(2006.01)*      *B01D 53/02* *(2006.01)*
*B01D 24/46* *(2006.01)*

(86) Internationale Anmeldenummer:
**PCT/EP2003/000298**

(87) Internationale Veröffentlichungsnummer:
**WO 2003/058009 (17.07.2003 Gazette 2003/29)**

(54) **VERFAHREN ZUM ENTKEIMEN UND REINIGEN VON WASSERFÜHRENDEN SYSTEMEN, INSBESONDERE IN SCHWIMM- UND BADEBECKENANLAGEN, UND VORRICHTUNG FÜR DESSEN DURCHFÜHRUNG**

METHOD FOR STERILISATION AND CLEANING OF WATER SUPPLY SYSTEMS, IN PARTICULAR IN SWIMMING AND BATHING POOL UNITS AND DEVICE FOR CARRYING OUT THE SAME

PROCEDE DE DEGERMAGE ET DE NETTOYAGE DE SYSTEMES D'ALIMENTATION EN EAU, NOTAMMENT DANS LES INSTALLATIONS DE PISCINES ET BASSINS DE BAIN, ET DISPOSITIF CORRESPONDANT

(84) Benannte Vertragsstaaten:
**AT BE BG CH CY CZ DE DK EE ES FI FR GB GR HU IE IT LI LU MC NL PT SE SI SK TR**

(30) Priorität: **14.01.2002 DE 10201089**

(43) Veröffentlichungstag der Anmeldung:
**20.10.2004 Patentblatt 2004/43**

(73) Patentinhaber: **P & W Invest Vermögensverwaltungsgesellschaft MbH 5020 Salzburg (AT)**

(72) Erfinder: **POLAK, Walter A-5020 Salzburg (AT)**

(74) Vertreter: **Hagemann, Heinrich et al Meissner, Bolte & Partner Postfach 86 03 29 81630 München (DE)**

(56) Entgegenhaltungen:
| | |
|---|---|
| DE-A- 1 434 837 | DE-A- 3 229 219 |
| DE-A- 3 403 631 | DE-A- 10 010 255 |
| DE-A- 10 028 090 | DE-C- 3 744 355 |
| FR-A- 2 676 218 | US-A- 6 139 756 |

**Beschreibung**

Die Erfindung betrifft ein Verfahren zum Entkeimen und Reinigen von wasserführenden Systemen, insbesondere in Schwimm- und Badebeckenanlagen, sowie eine Vorrichtung für dessen Durchführung.

Bei der Aufbereitung von Wasser, insbesondere in Schwimmbädern, müssen besonders hohe Anforderungen hinsichtlich der Aufbereitung und Desinfektion erfüllt werden. Die Hygiene des Schwimm- und Badebeckenwassers ist von ganz entscheidender Bedeutung. So spielen beispielsweise auch biogene Ablagerungen (Biofilme) - welche zwangsläufig in wasserführenden Systemen entstehen - im Filterbett sowie Kontaminationen des wasserführenden Systems (Rohrleitungen) und der Filter mit unterschiedlichsten Keimen, wie pathogenen Erregern, Sporenbildnern, Mikroorganismen und dergleichen, eine Rolle, so dass zur Entfernung dieser eine wirkungsvolle und technisch gut durchführbare Reinigung und Desinfektion - über die erforderliche Desinfektion des Wassers bzw. Schwimm- und Badebeckenwassers hinaus - in regelmäßigen Zeitabständen erfolgen muss.

Daher muss für eine gleichbleibend hohe Wasserqualität gesorgt werden, wobei einerseits eine ausreichend keimtötende Kapazität zum Desinfizieren bzw. Abtöten der Keime und Mikroorganismen bereitgestellt, aber gleichzeitig der Badegast in keiner Weise durch chemische oder mikrobiologische Belastungen des Badewassers beeinträchtigt werden darf.

Aus unsachgemäßer Wasseraufbereitung und -desinfektion können, z. B. durch pathogene Keime, Sporenbildner oder Viren etc., erhebliche Infektions- und damit Gesundheitsrisiken für den Badenden resultieren.

Untersuchungen haben gezeigt, dass etwa 80 bis 90% der unterschiedlichen Filtermaterialien, insbesondere im unteren Bereich, in hohem Maße durch Keime - auch humanpathogene, wie z. B. Pseudomonas aeruginosa oder Legionella pneumophilia - belastet sind. Neben dem Filtermaterial sind davon auch die Filtratkammerflächen, die Filterdüsen sowie die gesamte Verrohrung des wasserführenden Systems einer Schwimm- und Badebeckenanlage betroffen.

Bei bekannten Verfahren der Wasseraufbereitung und -desinfektion besteht der Nachteil, dass zusammen mit der üblichen Desinfektion des Badewassers die wasserführenden Systeme nur ungenügend gereinigt und desinfiziert werden. Selbst bei hohen Chlormengen ist dies der Fall. Die Nachteile hierfür liegen auf der Hand, so dass selbst bei hohen Konzenrationen eine zunehmende Belastung durch Wasserinhaltsstoffe und somit biogene Ablagerungen (Biofilme) der wasserführenden Systeme nicht vermieden werden können.

Es ist mittlerweile in der Fachwelt bekannt, dass durch die Biofilme ein eigener Lebensraum für Mikroorganismen entsteht, der dementsprechend gut mit Wasser, Substraten und Nahrung versorgt wird. Es leben in diesem "eigenen Lebensraum" verschiedene Mikrooragnismen, wie Bakterien, Protozoen oder Viren. Die Mikrooranismen haben in dieser Lebensgemeinschaft nicht nur Nahrung, sondern auch Schutz für weitere Entwicklung. Im Biofilm befinden sich dementsprechende Kanäle für die Versorgung der Mikroorganismen (Bewohner).

Das Gefährliche dabei ist, dass sich die Bakterien selbst aktiv lösen können und dann in Agglomeratform in das Wasser gelangen. Diese Agglomerate sind relative unempfindlich gegenüber Desinfektionsmittelgehalten, wie sie z. B. im Schwimm- und Badebeckenwasser vorgeschrieben sind.

Besonders gefährlich sind dann Bereiche ohne Desinfektionsmittel, wie sie in Schwimmbadanlagen - bei Einsatz von kohlehaltigen absorptiven Materialien - im unteren Bereich des Filtermaterials sowie der Filtratkammer und der Filtratleitung vorkommen. Aus diesen Gründen kommt es relativ rasch zu unerwünschtem Aufwuchs an Biofilmen.

Nicht zuletzt spielen die Rohmaterialien, welche vorwiegend aus Kunststoff sind, eine nicht unerhebliche Rolle.

Je älter diese Biofilme sind, desto schwieriger wird deren Abbau. Durch die üblicherweise eingesetzten Chlorgehalte, in z. B. Schwimmbädern werden bestehende Biofilme nicht entfernt. Der Abbau von Biofilmen mittels geeigneter Verfahren wird aufgrund der mittlerweile erkannten Risiken für die menschliche Gesundheit zukünfig noch mehr Bedeutung gewinnen.

Nach einem Beispiel aus dem Stand der Technik beschreibt die DE-32 29 219 die Verwendung einer grenzflächenaktiven Substanz als Rückspülmittel für partikelhaltige rückspülbare Filterbetten zur Wasseraufbereitung bei Schwimmbad-, Trink- und Abwasseraufbereitungsanlagen. Bei diesem Verfahren ist die Reinigungswirkung gegenüber festen Ablagerungen jedoch nur mäßig. Insbesondere wird nur eine geringe Wirkung gegenüber Kontaminationen des Filters mit Mikroorganismen erzielt. Darüber hinaus muss die grenzflächenaktive Substanz in hohen Konzentrationen eingesetzt werden, was zu großen Kosten und zu einer hohen Belastung des Abwassers führt.

Ein Schwimmbecken, das mit einem Schwallwasserbehälter zur Regulierung des Wasservolumens in dem System über eine gemeinsame Leitung mit einer Umwälzpumpe und einem Filter verbunden ist, ist aus der DE-C1-37 44 355 bekannt. Die gemeinsame Leitung wird hierbei in einer Weise verwendet, dass eine druckabhängige Zumischung von Wasser aus dem Schwallwasserbehälter zum umgewälzten Beckenwasser erfolgt. Ein Reinigungs- und Desinfektionsverfahren sowie dafür erforderliche Vorrichtungsmerkmale sind nicht offenbart.

Die DE-A1-100 10 255 beschreibt ein zweistufiges Verfahren zum entkeimenden und reinigenden Spülen partikelhaltiger Filterbetten von zyklischen oder linearen Wasseraufbereitungsanlagen, wobei der Filter in einem ersten Verfahrensschritt mit einer Spüllösung beschickt und nach Einwirken der Spüllösung auf das Filtermaterial in einem zweiten Verfahrensschritt mit Wasser gespült wird. Dieses Verfahren ist auf die Reinigung und Desinfektion von Filtern beschränkt und ermöglicht nicht die wirksame Dekontamination weiterer Komponenten und insbesondere nicht des gesamten wasserführenden Systems. Auch wird keine entsprechende Vorrichtung beschrieben.

Die FR-A1-2 676 218 beschreibt eine Desinfektionsmittelmess- und -dosiereinrichtung für ein Schwimmbecken in paralleler Anordnung zu und verbunden mit einem geschlossenen Wasserkreislauf, aufweisend das Schwimmbecken, eine Pumpe und einen Filter, zur Aufrechterhaltung einer vorbestimmten Desinfektionswirkung im Schwimmbeckenwasser durch eine geregelte Zudosierung von Desinfektionsmittel in den geschlossenen Wasserkreislauf. Die offenbarte Mess- und Dossiereinrichtung ermöglicht keine wirksame Dekontamination einzelner Komponenten des wasserführenden Systems. Die Dekontamination des gesamten Systems ist zudem auf die Desinfektion des Schwimmbeckenwassers beschränkt.

Es war daher Aufgabe der vorliegenden Erfindung, ein Verfahren bereitzustellen, das die vorstehend geschilderten Nachteile nicht zeigt. Insbesondere sollte das erfindungsgemäße Verfahren neben einer hohen Reinigungs- und Entkeimungswirkung - zur Lösung der Biofilm-Problematik - auch die wirksame Dekontamination des gesamten wasserführenden Systems oder der gesamten wasserführenden Fläche, wie z.B. einer Schwimm- oder Badeanlage, ermöglichen, insbesondere Mikroorganismen, pathogene Keime, Sporenbildner, Viren, Pilze sowie Kontaminationen aller Art beseitigen. Ferner sollte das Verfahren gleichzeitig kostengünstig sein, d.h. einen wirtschaftlichen Betrieb erlauben, und nur zu geringen Belastungen des Abwassers führen.

Erfindungsgemäß wird diese Aufgabe durch ein Verfahren zum Entkeimen und Reinigen von wasserführenden Systemen und Flächen, wie z.B. in Schwimm- und Badebeckenanlagen, mit den Schritten gelöst:

(1) Aufbauen eines geschlossen Kreislaufs zwischen zwei Punkten des wasserführenden Systems bzw. der wasserführenden Fläche,

(2) Zugeben einer vorbestimmten Menge eines desinfizierenden Wirkstoffs in Form einer chloroxid-, halogen- und/oder peroxidhaltigen wässerigen Spüllösung zum Entkeimen und Reinigen,

(3) Entkeimen und Reinigen der im System vorhandenen Rohre, Filter und Filtermaterialien, einschließlich Filterkohlen mit und ohne adsorptive Eigenschaften, unter periodischem Umwälzen der Spüllösung im geschlossenen Kreislauf in definierten Zeitintervallen,

(4) Ablassen und Verwerfen der wässerigen Spüllösung und

(5) Wiederherstellen des ursprünglichen Wasserlaufs.

Vorzugsweise wird das Verfahren zum Entkeimen und Reinigen einer Schwimm- oder Badebeckenanlage angewendet.

Nach dieser ersten erfindungsgemäßen Verfahrensvariante wird daher zunächst in Schritt (1) ein geschlossener Kreislauf zwischen dem Ablauf des Schwallwasserbeckens und dem Zulauf unmittelbar vor Eintritt des Reinwassers in das Schwimmbad- oder Badebecken aufgebaut. Dies kann je nach der vorhandenen Schwimm- oder Badebeckenanlage durch die dem Fachmann bekannten Mittel erfolgen und hängt von den jeweiligen baulichen Gegebenheiten ab. Hieran schließt sich in Schritt (2) das Zugeben einer vorbestimmten Menge eines desinfizierenden Wirkstoffs, ausgewählt aus einer chloroxid-, halogen- und/oder peroxidhaltigen wässerigen Spüllösung zum Entkeimen und Reinigen an. Die Menge der Spüllösung und deren Zusammensetzung können je nach Bedarf modifiziert werden, und sind anhand des vorliegenden wasserführenden Systems, dessen Größe, Fassungsvermögen, Verschmutzungsgrad sowie der zur Verfügung stehenden Zeit zur Reinigung entsprechend zu bestimmen.

In Schritt (3) erfolgt das Entkeimen und Reinigen der im System vorhandenen Rohre, Filter und Filtermaterialien, einschließlich Filterkohlen mit und ohne adsorptive Eigenschaften, unter periodischem Umwälzen der Spüllösung in dem geschlossenen Leitungssystem in definierten Zeitintervallen. Mit anderen Worten muss die Spüllösung nicht ständig umgewälzt werden, sondern es können auch längere Einwirkzeiten einbezogen werden, in denen nicht umgewälzt wird.

Es hat sich als zweckmäßig erwiesen, wenn die Spüllösung während weniger Minuten bis zu mehreren Stunden, zum Beispiel etwa 3 bis 10 Minuten, oder auch etwa 3 bis 12 h, bevorzugt etwa 4 bis 8 h, insbesondere etwa 4 bis 6 h, umgewälzt wird, wobei das Umwälzen ein oder mehrere Male unterbrochen werden kann. Das Umwälzen wird daher von sogenannten Ruhephasen abgelöst, in denen die Spüllösung innerhalb einer bestimmten Einwirkzeit stehen gelassen wird. Das Umwälzen wird vorzugsweise in eine Vorzugsrichtung oder kann abwechselnd in eine der beiden Richtungen durchgeführt werden.

Nach dem Entkeimen und Reinigen wird in Schritt (4) der ersten Verfahrensvariante der Erfindung die wässerige Spüllösung abgelassen und verworfen. Die Spüllösung kann nach Prüfung auf Vorhandensein von noch nicht verbrauchtem Wirkstoff (qualitativer Test) mehrfach zum Einsatz kommen.

Schließlich wird in Schritt (5) durch einfachen Abbau oder Entfernen der zusätzlichen übergangsweise bereitgestellten Leitungen, Vorrichtungen oder Systeme der ursprüngliche Wasserlauf der Schwimm- und Badeanlage hergestellt, so dass der normale Betrieb wieder aufgenommen werden kann.

Es empfiehlt sich, diese erste Verfahrensvariante mit den Schritten (1) bis (5) zu wiederholen, d.h. in regelmäßigen Zeitintervallen durchzuführen, um eine entsprechende Reinigungs- und Entkeimungswirkung beizubehalten bzw.

für einen längeren Zeitraum einer unerwünschten Kontamination vorzubeugen.

Bei einer weiteren bevorzugten Ausführungsform handelt es sich bei dem wasserführenden System um ein Wanrmsprudelbecken, insbesondere ein Hot-Whirl-Pool oder eine Whirl-Wanne. Das Verfahren kann jedoch auch mit gutem Ergebnis in einem Trinkwasser-Zuführungssystem in einer Hausinstallation angewendet werden, insbesondere in einem Dusch-System. Bei Anwendung in einem Dusch-System ist besonders bevorzugt, dass der Aufbau eines geschlossenen Kreislaufs in Schritt (1) zwischen dem Warmwasserboiler und dem Duschkopf erfolgt. Gute Ergebnisse werden ferner erzielt, wenn das Verfahren auf ein Prozesswasser-Aufbereitungssystem im industriellen Bereich angewendet wird.

Besonders vorteilhaft ist auch eine weitere erfindungsgemäße Verfahrensvariante, wonach ein Verfahren zum Entkeimen und Reinigen von wasserführenden Systemen und Flächen in Aufbereitungsanlagen, Rohrsystemen sowie Schwimm- und Badebeckenanlagen mit den Schritten bereitgestellt wird:

(a) Abtrennen eines definierten Abschnitts innerhalb des wasserführenden Systems,
(b) Einbringen einer hochkonzentrierten Spüllösung, enthaltend zumindest einen desinfizierenden Wirkstoff,
(c) Durchspülen des definierten Abschnitts mit der Spüllösung, gegebenenfalls unter Umwälzen,
(d) Sammeln der Spüllösung,
(e) Überprüfen des Vorhandenseins des Wirkstoffs und/oder der Konzentration des Wirkstoffs und/oder der Entkeimungs- und Reinigungswirkung,
(f) gegebenenfalls Wiederholen der Schritte (c) bis (e),
(g) Ablassen und Verwerfen der wässerigen Spüllösung und
(h) Wiederherstellen des ursprünglichen Wasserlaufs der Schwimm- und Badebeckenanlage.

Nach dieser erfindungsgemäßen zweiten Variante wird zunächst in Schritt (a) ein definierter Abschnitt im wasserführenden System abgetrennt, d.h. ein bestimmter Bereich ausgewählt und zwei Abgrenzungen vorgesehen. In diesen definierten Bereich wird dann gemäß Schritt (b) eine hochkonzentrierte Spüllösung, enthaltend zumindest einen desinfizierenden Wirkstoff, eingebracht.

Daraufhin erfolgt das Durchspülen des definierten Abschnitts mit der Spüllösung (Schritt (c)). Den Ausdruck "Durchspülen" versteht man in der vorliegenden Erfindung hierbei weitestgehend. Dies kann entsprechend einer Möglichkeit durch Einbringen von Gas und Bewegen der Spüllösung durch den definierten Abschnitt unter Einsatz von Druck, erfolgen. Vorzugsweise wird Druckluft verwendet. Nach einer weiteren Ausführungsform können die Rohrinnenwände, Filter und Filtermaterialien mit der Spüllösung auch besprüht werden. Optional kann die Spüllösung auch wie oben geschildert umgewälzt werden.

Nach dem Sammeln der Spüllösung in Schritt (d) wird überprüft, ob noch desinfizierender Wirkstoff vorhanden ist. Anhand der verbrauchten Menge des Wirkstoffs (auch Zehrung genannt) kann auf die desinfizierende Wirkung rückgeschlossen werden. Eine vollständige Desinfektion liegt dann vor, wenn kein Desinfektionsmittel mehr verbraucht wird. Eine zusätzliche, genauere Überprüfung kann durch die Bestimmung der Konzentration des Wirkstoffs vor und nach der desinfizierenden Spülung und anschließendem Vergleich beider Konzentrationswerte erfolgen. Je nach Bedarf kann eine entsprechende Methode zur Überprüfung der Desinfektionswirkung gewählt werden.

Erfindungsgemäß erfolgt das Überprüfen der Entkeimungs- und Reinigungswirkung vorzugsweise durch den bekannten Bart-Test (biological activity reaction test) und kann je nach vorhandenen Filtermaterialien auch durch weitere bekannte Tests betreffend die Adsorptionsaktivität bzw. weitere biologische Untersuchungen erfolgen. Derartige Überprüfungsverfahren, Schnelltests und dergleichen sind dem Fachmann bekannt, so dass auf detaillierte Erläuterungen verzichtet werden kann.

Je nach Bedarf können die Schritte (c) bis (e) zum Entkeimen und Reinigen des wasserführenden Systems wiederholt werden.

Erfindungsgemäß ist es auch möglich die oben geschilderte erste und zweite Verfahrensvarianten zu koppeln, so dass besonders schwierig zu reinigende Abschnitte einer Prozessstrecke zusätzlich einer gesonderten Reinigung und Desinfektion unterzogen werden können. Beispielsweise ist es bei der Reinigung und Desinfektion von Prozessstrekken, bei denen die mittlere Aufbereitungsstufe - die Filtration - mit Filtermaterialien, welche adsorptive Eigenschaften haben (z.B. Aktivkornkohlen oder bestimmte Braunkohlesorten), erfolgt, von Vorteil, dieses Filtermaterial zunächst gesondert zu behandeln, indem man die erforderliche Menge an Spüllösung, welche zur Reinigung (chemisch und mikrobiologisch) von kohlehaltigen Materialien mit adsorptiven Eigenschaften benötigt wird, als erstes einbringt und einwirken läßt (erste Verfahrensvariante der Erfindung).

Anschließend kann dann der gesamte Reinigungsprozess mittels Umwälzung der Spüllösung entweder in nur einem Teil einer festgelegten Prozessstrecke durchgeführt werden (Wiederholung der ersten Variante der Erfindung) oder die gesamte Prozessstrecke kann der Reinigung und Desinfektion unterzogen werden (zweite Verfahrensvariante der Erfindung).

So ist es auch möglich, periodisch z.B. einen unerwünscht hoch verkeimten Aktivkornkohlefilter, welcher zum Beispiel einem Sandfilter nachgeschaltet ist, mit diesem Reinigungssystem speziell zu behandeln. Mit dem Verfahren der Erfindung können somit in einer Prozessstrecke gezielt ganz bestimmte Abschnitte einer Verrohrung, wie z.B. die Filtrat- und Reinwasserleitung, gereinigt und desinfiziert werden.

Nach einer besonders bevorzugten Ausführungsform der Erfindung wird als wässerige Spüllösung eine chloroxidhaltige wässerige Lösung eingesetzt. Es hat sich gezeigt, dass unter den Chloroxiden sowohl aus Gründen der Wirksamkeit, als auch der Zugänglichkeit das Chlordioxid besonders bevorzugt ist. Da es sich bei Chlordioxid um eine chemisch instabile und schwer handhabbare Substanz handelt, ist es vorteilhaft, dass ein großer Teil des Chlordioxids in der Lösung nicht in freier Form vorliegt, sondern chemisch gebunden in Form einer chemischen Spezies, aus der Chlordioxid stetig nachgebildet wird. Eine solche Substanz ist das Tetrachlordecaoxid-Komplex-Dianion $[Cl_4O_{10}]^{2-}$. Dieses Komplex-Dianion, das unter der ELINCS-Nummer 420-970-2 dokumentiert ist, reagiert in wässeriger Lösung im Sinne folgender beider Reaktionen:

$$(1) \quad [Cl_4O_{10}]^{2-} \quad \rightleftharpoons \quad 4\,ClO_2 + O_2 + 2e^- \quad \text{(Durchlaufphase)}$$

$$(2) \quad [Cl_4O_{10}]^{2-} + 2e^- \quad \longrightarrow \quad 5\,O_2 + 4\,Cl^- \quad \text{(Bilanz Bruttogleichung)}$$

Das Tetrachlordecaoxid-Komplex-Dianion steht also mit dem Chlordioxid in einem Gleichgewicht, so dass die Lösung des Dianions stets eine gewisse Menge an Chlordioxid enthält, das bei Zerfall des Komplexes als Zwischenprodukt auftritt und bei dessen Verbrauch nachgebildet wird. Der TCDO-Komplex stellt daher einen biokatalytisch aktivierbaren Sauerstoffträger dar, der sich hinsichtlich seiner Oxidationseigenschaften qualitativ von Hypochlorit, Chlorat und Wasserstoffperoxid sowie quantitativ von Chlorit unterscheidet. Als Endreaktionsprodukte werden lediglich Sauerstoff und Chlorid gebildet, welche auch zu keinen Abwasserproblemen führen.

In einer bevorzugten Ausführungsform der Erfindung wird demzufolge eine wässerige Lösung des Tetrachlordecaoxid-Komplex-Dianions $[Cl_4O_{10}]^{2-}$ eingesetzt, die aufgrund des biokatalytisch selektiven Wirkungsmechanismus herausragende Eigenschaften aufweist. Diese Tetrachlordecaoxid-Komplex-Dianionen enthaltende wässerige Lösung wird vorzugsweise erhalten, indem eine einen pH-Wert $\leq 3$ aufweisende sulfationenhaltige, wässerige Lösung mit einer darin stabilen Peroxoverbindung versetzt, und diese Lösung anschließend mit der alkalischen, wässerigen Lösung eines Chlorits vermischt wird. Bevorzugt wird dabei die sulfationenhaltige, wässerige Lösung mit einer solchen Menge einer darin stabilen Peroxoverbindung versetzt, dass sich eine Konzentration an Peroxoverbindung in der Lösung von etwa 0,001 bis 0,01 molar ergibt. Erfindungsgemäß ist es auch möglich, dass die sulfationenhaltige, wässerige Lösung mit einer darin stabilen Peroxoverbindung versetzt und diese Lösung anschließend mit der alkalischen, wässerigen Lösung eines Chlorits in einer Menge vermischt wird, so dass sich ein pH-Wert von über 7,0, insbesondere zwischen 7,5 und 8,0, einstellt.

Als besonders vorteilhaft hat es sich erwiesen, als Peroxoverbindung eine anorganische Peroxoverbindung in Form von Wasserstoffperoxid, einem Persulfat, Percarbonat, Perborat oder einem Peroxid eines Alkali- oder Erdalkalimetalls zu verwenden. Als Chlorit wird vorzugsweise ein Alkali- und/oder Erdalkalichlorit eingesetzt. Besonders vorteilhafte Ergebnisse stellen sich dann ein, wenn die sulfationenhaltige, wässerige Lösung einen pH-Wert $\leq 1$ aufweist.

Wenn die eingesetzten wässerigen Ausgangslösungen lediglich eine geringe Carbonathärte zeigen bzw. mit demineralisiertem Wasser hergestellt werden, empfiehlt es sich, unter einer Inertgasatmosphäre zu arbeiten, da es in Abwesenheit einer Schutzgasschicht zwischen dem geringfügig ausgasenden Chlordioxid und Luft zur Bildung von explosiven Luft/Chlordioxidgemischen kommen kann. Luft/Chlordioxidgemische neigen bei einem Verhältnis der Bestandteile Luft zu Chlordioxid von etwa 10:1 zu einem explosionsartigen Zerfall des Chlordioxids in Chlor und Sauerstoff. Bei wässerigen Ausgangslösungen mittlerer bzw. hoher Carbonathärte (> etwa 7 Grad bzw. > etwa 1,3 mmol/l Carbonathärte) ist der Einsatz eines Inertgases im allgemeinen nicht nötig, da sich eine Art Schutzgasschicht aus Kohlendioxid bildet. Vorzugsweise wird daher zur Herstellung der sulfationenhaltigen wässerigen Lösung, die zur Bildung der Tetrachlordecaoxid-Komplex-Dianionen enthaltenden Lösung eingesetzt werden soll, mineralisiertes Wasser verwendet.

Vorzugsweise enthält die Tetrachlordecaoxid-Komplex-Dianionen enthaltende Lösung ein wasserlösliches Phosphat, da die Menge an Peroxoverbindungen herabgesetzt werden kann, wenn der Fertiglösung ein wasserlösliches Phosphat, so z.B. Natriummetapolyphosphat, in geringer Menge einverleibt wird.

Die Konzentration, mit der die wässerige Spüllösung verwendet wird, hängt von verschiedenen Faktoren ab, wie beispielsweise dem Grad der Verschmutzung und/oder biogenen Ablagerungen bzw. der bakteriellen Kontamination des wasserführenden Systems. Vorteilhafterweise wird die chloroxid-, halogenhaltige und/oder peroxidhaltige wässerige Spüllösung in dem erfindungsgemäßen Verfahren daher in einer Konzentration, die einer Anfangskonzentration an Chlorit von mindestens etwa 0,1 Mol/l, bevorzugt mindestens etwa 0,15 Mol/l, entspricht, eingesetzt. Dies hat erfindungsgemäß zu besonders guten Ergebnissen geführt.

Selbstverständlich können neben den oben beschriebenen erfindungsgemäßen Varianten der wässerigen Lösung des Tetrachlordecaoxid-Komplex-Dianions $[Cl_4O_{10}]^{2-}$ andere chloroxid-, halogenhaltige und/oder peroxidhaltige wässerige Spüllösungen zum Einsatz kommen, die jedoch weniger bevorzugt sind.

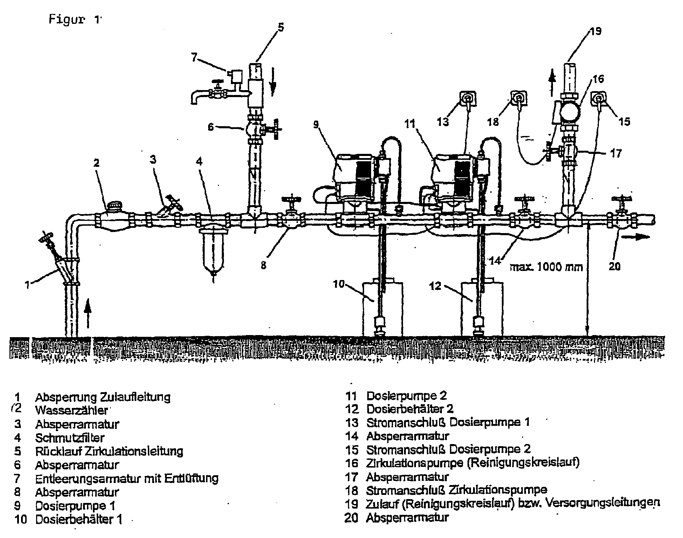

Das Verfahren kann auch so ausgelegt werden, dass eine ständige Zudosierung von Desinfektionsmitteln vorgesehen ist. Eine solche Dosiertechnik kann z.B. durch Zulaufwasser einer Anlage zur Bereitstellung von Wasch- und Duschwasser, aber auch im Bypass einer Rohrwasserleitung von Schwimmbadanlagen bzw. in Zulaufleitungen von Wasser für industrielle Zwecke zum Einsatz kommen. Besonders bevorzugt ist es, eine Anlage so auszulegen, dass sowohl eine periodische Spülung des Desinfektionsmittels als auch eine kontinuierliche Zudosierung des Desinfektionsmittels möglich ist. Fig. 1 zeigt eine Anordnung für eine Duschanlage, mit der es sowohl möglich ist, die nötigen Desinfektionsmittelmengen für periodische Spülungen von wasserführenden Systemen einzubringen, aber auch eine ständige Zudosierung von Desinfektionsmitteln durchgeführt werden kann.

Für den Fall der periodischen Spülung werden die Absperrarmaturen (3) und (20) geschlossen. Der Entleerungshahn (7) wird geöffnet, und es wird so viel Wasser aus dem System entnommen, wie anschließend mit der Desinfektionsmittellösung-Gebrauchslösung wieder aufgefüllt wird. Die Absperrarmaturen (6) und (17) müssen vor der Entleerung und Füllung mit der Desinfektionsmittellösung geöffnet sein. Anschließend wird die Zirkulationspumpe (16) in Betrieb gesetzt und gleichzeitig die erforderliche Menge des Desinfektionsmittels zudosiert (die Spülung erfolgt nach einem Stop-and-Go-Verfahren, wobei die Förderstromrichtung in bestimmten Intervallen geändert wird). Nach dem Spülvorgang wird das gesamte System entleert. Anschließend wird das System wiederum mit Trinkwasser gefüllt, nochmals zirkuliert und entleert. Somit ist sichergestellt, dass eventuell noch vorhandene Desinfektionslösung und Reste von biogenen Ablagerungen und Verunreinigungen aus dem System herausgespült werden. Des weiteren wird die Leitung wiederum mit Trinkwasser befüllt und kann wieder in Betrieb gehen.

Falls eine kontinuierliche Zudosierung des Desinfektionsmittels vorgenommen werden soll, werden die beiden Absperrarmaturen (6) und (20) geschlossen. Die Absperrarmaturen (1), (3), (8), (14) und (17) sind geöffnet. Mit den beiden Dosierpumpen und Kontaktwasserzählern wird die erforderliche Menge an Desinfektionsmittellösung abhängig vom Wasserverbrauch zudosiert.

So wie hier am Beispiel einer Duschanlage beschrieben, sind weitere Anwendungsmöglichkeiten des Verfahrens zum Entkeimen und Reinigen von wasserführenden Systemen sowie eine mengenproportionale Dosierung von Desinfektionsmittel möglich.

Gegenstand der Erfindung ist auch eine Vorrichtung zum Entkeimen und Reinigen von wasserführenden Systemen und Flächen, z.B. in Schwimm- und Badebeckenanlagen, umfassend:

(1) einen geschlossen Kreislauf zwischen zwei Punkten des wasserführenden Systems bzw. der wasserführenden Fläche, enthaltend eine vorbestimmte Menge zumindest eines desinfizierenden Wirkstoffs in Form einer chloroxid-, halogen- und/oder per-oxidhaltigen wässerigen Spüllösung zum Entkeimen und Reinigen,
(2) eine Pumpe zum Umwälzen der Spüllösung im geschlossenen Leitungssystem unter Entkeimen und Reinigen der im System vorhandenen Rohre und Filter,
(3) einen Ablaß für die wässerige Spüllösung und
(4) Mittel zur Wiederherstellung des ursprünglichen Wasserlaufs.

Bei der bevorzugten Ausführungsform handelt es sich bei dem wasserführenden System um eine Schwimm- oder Badebeckenanlage. In diesem Fall erfolgt das Entkeimen und Reinigen der Schwimm- und Badebeckenanlage durch eine Vorrichtung, umfassend:

(1) einen geschlossenen Kreislauf zwischen dem Ablauf des Schwallwasserbeckens und dem Zulauf unmittelbar vor Eintritt des Reinwassers in das Schwimm- und Badebecken, enthaltend eine vorbestimmte Menge eines desinfizierenden Wirkstoffs in Form einer chloroxid-, halogen- und/oder peroxidhaltigen wässerigen Spüllösung zum Entkeimen und Reinigen,
(2) eine Pumpe zum Umwälzen der Spüllösung im geschlossenen Kreislauf unter Entkeimen und Reinigen der im System vorhandenen Rohre und Filter,
(3) einen Ablass für die wässerige Spüllösung und
(4) Mittel zum Wiederherstellen des ursprünglichen Wasserlaufs.

Die erfindungsgemäße Vorrichtung umfasst somit einen geschlossenen Kreislauf zwischen dem Ablauf des Schwallwasserbeckens und dem Zulauf unmittelbar vor Eintritt des Reinwassers in das Schwimm- und Badebecken, und enthält eine vorbestimmte Menge einer chloroxid-, halogen- und/oder peroxidhaltigen wässerigen Spüllösung zum Entkeimen und Reinigen. Hierbei handelt es sich vorzugsweise um das bereits beschriebene Tetrachlordecaoxid-Komplex-Dianion in wässeriger Lösung.

Weiterhin ist zumindest eine Pumpe zum Umwälzen der Spüllösung im geschlossenen Kreislauf unter Entkeimen und Reinigen der im System vorhandenen Rohre und Filter vorgesehen, welche im sogenannten "stop and go"-Modus arbeitet, d.h. je nach Bedarf ein definiertes Zeitintervall umwälzt, wobei die Umwälzung auch abwechselnd einmal in die eine und dann in die andere Richtung erfolgen kann, und anschließend abschaltet, damit die Spüllösung im wasserführenden System stehen bleibt und einwirken kann, um dann erneut wieder mit dem Umwälzen der Spüllösung zu beginnen.

Nach einer bevorzugten erfindungsgemäßen Weiterbildung kann die Vorrichtung zum Entkeimen und Reinigen von wasserführenden Systemen und Flächen in Aufbereitungsanlagen, Rohrsystemen sowie Schwimm- und Badebeckenanlagen umfassen:

(a) Mittel zum Abtrennen eines definierten Abschnitts innerhalb des wasserführenden Systems,
(b) Mittel zum Einbringen einer hochkonzentrierten Spüllösung, enthaltend zumindest einen desinfizierenden Wirkstoff,
(c) Mittel zum Durchspülen des definierten Abschnitts mit der Spüllösung,
(d) eine Vorrichtung zum Sammeln der Spüllösung,
(e) Mittel zum Überprüfen des Vorhandenseins des Wirkstoffs und/oder der Konzentration des Wirkstoffs und/oder der Entkeimungs- und Reinigungswirkung,
(f) Mittel zum Ablassen der wässerigen Spüllösung und
(g) Mittel zum Wiederherstellen des ursprünglichen Wasserlaufs der Schwimm- und Badebeckenanlage.

Dies erlaubt eine optimale Steuerung und Kontrolle der Reinigungs- und Entkeimungswirkung des wasserführenden Systems, wie bereits bei der entsprechenden Verfahrensvariante der Erfindung eingehend erläutert.

Das Verfahren sowie die Vorrichtung der Erfindung eignen sich zum Entkeimen und Reinigen verschiedener Typen von wasserführenden Systemen in Badeanlagen, insbesondere Schwimm- und Badebeckenkreisläufe, wobei keine prinzipiellen Einschränkungen hinsichtlich der Art, Größe und des Aufbaus des wasserführenden Systems bestehen. Zusätzlich können die beiden Verfahrensvarianten bzw. Vorrichtungen der Erfindung kombiniert werden, wodurch auch neuralgische Bereiche der Prozessstrecke entkeimt und gereinigt werden können.

Zusammenfassend läßt sich feststellen, dass erfindungsgemäß ein Entkeimen und Reinigen von Schwimmbadanlagen ermöglicht wird, das im Vergleich zum Stand der Technik zu stark verbesserten Ergebnissen führt. So ist die Reinigungswirkung gegenüber biogenen Ablagerungen (Biofilmen) deutlich verbessert. Besonders ausgeprägt sind dadurch die sehr entscheidenden Vorteile hinsichtlich der Wirkung gegenüber Kontaminationen durch Keime, Mikroorganismen, Sporenbildner, Viren und Pilze. Die im Stand der Technik beschriebenen Reinigungsverfahren erzielen hier nur eine schwache Wirkung. Überraschenderweise hat sich gezeigt, dass das erfindungsgemäße Verfahren nicht nur zu einer weitgehenden Dekontamination bezüglich eines Befalls an Mikroorganismen bzw. des Aufbaus von Biofilmstrukturen führt, sondern auch eine starke vorbeugende Wirkung erzielt wird. Dies ist möglicherweise darauf zurückzuführen, dass durch das Abtöten sämtlicher Keime und die gleichzeitige Beseitigung nahezu aller Verunreinigungen, die das Wachstum der Mikroorganismen fördern könnten, indem sie z.B. als Nahrungsquelle dienen, ein Ausbreiten von Mikroorganismen auch für einen längeren Zeitraum nach der Behandlung verhindert wird. Der Einsatz des erfindungsgemäßen Verfahrens kann und sollte daher als vorbeugende Maßnahme zum aktiven und vorbeugenden Gesundheitsschutz gesehen und durchgeführt werden.

Durch die oben geschilderten erfindungsgemäßen Ausführungsformen, ist es daher möglich, gezielt bestimmte Abschnitte einer Prozessstrecke auszuwählen und das Reinigungs- und Desinfektionsverfahren hierauf abzustimmen. Dies bewirkt einen geringeren Verbrauch an Wasser, insbesondere bei relativ großen Rohrquerschnitten. Die Menge der notwendigen Spüllösung kann drastisch reduziert werden, wobei gleichzeitig dieselbe hohe Reinigungs- und Entkeimungswirkung erzielt wird. Ein weiterer Vorteil der geschilderten Verfahrensvarianten der Erfindung erlaubt die Kontrolle der Reinigungswirkung nach jedem Durchlauf, so dass eine gezielte Steuerung erreicht wird und nur wenn nötig ein weiterer Durchlauf erfolgt. Dies stellt sicher, dass die Konzentration der Spüllösung, die Dauer des Reinigungsvorgangs und die eingesetzte Wassermenge optimal aufeinander abgestimmt werden. Darüber hinaus können auch besonders schwierig zu reinigende Regionen erfasst werden, in einer Prozessstrecke können gezielt bestimmte Abschnitte einer Verrohrung, wie z.B. die Filtrat- und Reinwasserleitung, gereinigt und desinfiziert werden. Somit können erfindungsgemäß aufgrund der deutlich verringerten Wassermenge bei der Spüllösung Kosten eingespart werden, wobei zudem keine nennenswerte Belastung des Abwassers auftritt.

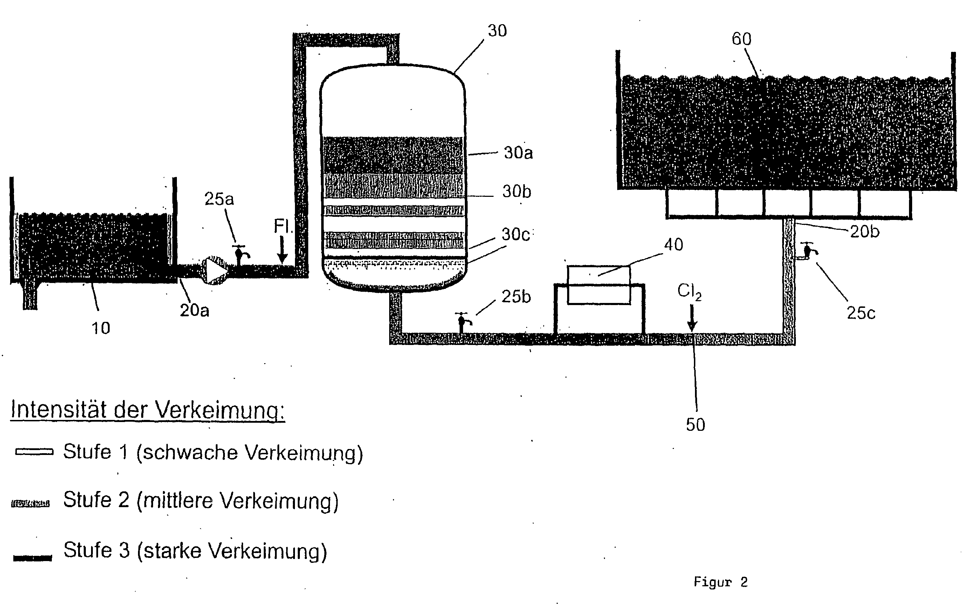

Eine Ausführungsform der erfindungsgemäßen Vorrichtung wird anhand der beigefügten Figur 2 gezeigt. Hier sind schematisch dargestellt:

- Schwallwasserbecken (10),
- Zugang bei der Rohwasserverrohrung (20a),
- Probeentnahmeleitung und -entnahmehahn (25a) für Rohwasser vor der ersten Aufbereitungsstufe der Flockung (Fl.),

- Filter (30) mit

  - Adsorptionsstufe (30a)
  - Sperrschicht (30b) und
  - Stützschichten (30c),

- Probeentnahmeleitung und -entnahmehahn (25b) im Filtrat vor der Desinfektion (Chlorung),
- Wärmetauscher (40),
- Vorrichtung für die Basis- und Betriebschlorung (50),
- Probeentnahmeleitung und -entnahmehahn (25c) im Reinwasser nach der Desinfektion (Chlorung) unmittelbar vor Eintritt in das Becken,
- Zugang bei der Reinwasserverrohrung (20b) und
- Schwimmbecken (60).

Der Filter (30) zeigt im unteren Bereich, d.h. in den Stützschichten (30c), den stärksten Verkeimungsgrad, der sich dann bis zur Vorrichtung für die Basis- und Betriebschlorung (50) fortsetzt und in abgeschwächter Form bis zum Schwimmbecken (60) reichen kann. Jedoch auch im oberen Bereich des Filters, der Adsorptionsstufe (30a), und an den Beckenrändern des Schwallwasserbeckens (10) treten starke bis mittelstarke Verkeimungen auf.

Um diese besonders stark verkeimten Bereiche der Prozeßstrecke im wasserführenden System der Schwimmbadanlage zu entkeimen und zu reinigen, wird daher zwischen dem Ablauf des Schwallwasserbeckens, d.h. dem Zugang (20a) und dem Zulauf unmittelbar vor Eintritt des Reinwassers in das Schwimm- oder Badebecken, dem Zugang (20b), ein geschlossenes Leitungssystem gebildet (nicht gezeigt). Diesem System wird eine vorbestimmte Menge einer chloroxid-, halogen- und/oder peroxidhaltigen wässerigen Spüllösung zum Entkeimen und Reinigen, wie bevorzugt der TCDO-Komplex, zugegeben und unter Verwendung einer Pumpe (nicht gezeigt) in definierten Zeitintervallen umgewälzt. Während dem Umwälzen der Spüllösung sollten die Probeentnahmehähne leicht geöffnet werden, um ebenfalls eine Reinigung und Desinfektion der Rohre und Schläuche, welche sich zwischen den Rohrleitungen (Rohwasser-, Filter und Reinwasserleitung) und den Probeentnahmehähne befinden, durch die Spüllösung zu ermöglichen.

Dies ist insofern wichtig, da sich bekanntermaßen im Laufe der Zeit und während des Betriebes der Umwälzanlage (Filtrationsvorgang) das Wasser in diesen Rohr- und Schlauchleitungen überwiegend in einer Stagnationsphase befindet. Dadurch kommt es vielfach zu einer verstärkten mikrobiologischen Kontamination der Rohre und Schläuche der Probeentnahmehähne und zu einer möglichen Fehlinterpretation bei der mikrobiologischen Untersuchung.

Nach erfolgter Reinigung und Entkeimung wird die wässerige Spüllösung abgelassen und der in Figur 1 gezeigte ursprüngliche Wasserlauf der Badanlage wieder hergestellt.

Nachfolgend soll die Erfindung anhand von Beispielen veranschaulicht werden:

**Beispiel 1:**

- Herstellung einer Tetrachlordecaoxid-Komplex-Dianionen enthaltenden Lösung -

In 11 sulfathaltiges Wasser (Carbonathärte: 18 Grad bzw. 3,2 mmol/l) eines pH-Wertes von 0,5 werden 0,5 g einer 30 Gew.-%igen Wasserstoffperoxidlösung gegeben. Zu dieser Lösung werden unter gutem Rühren 0,9 1 einer handelsüblichen Natriumchloritlösung (etwa 300 g Natriumchlorit/l) hinzugefügt. Dabei durchläuft die Lösung eine braune Färbung, die beim Überschreiten des pH-Wertes von 7 nach Ablauf der Stabilisientgsreaktion in eine helle lindgrüne Farbe umschlägt. Hierbei stellt sich in der Lösung ein pH-Wert von etwa 7,5 ein.

**Beispiel 2:**

- Entkeimung und Reinigung des wasserführenden Systems eines Schwimmbads mit der ersten erfindungsgemäßen Verfahrens- bzw. Vorrichtungsvariante -

Im vorliegenden Fall wurde eine Badewasseraufbereitungsanlage eines Schwimmer- und Nichtschwimmerbeckens in einem öffentlichen Hallenbad entkeimt und gereinigt. Der Förderstrom beträgt Qh 150 m$^3$ bei einem Beckenwasservolumen von ca. 470 m$^3$. Die Badeanlage wird von durchschnittlich 350 Personen/Tag genutzt. Die Besucherfrequenz während der Betriebszeiten bewegt sich zwischen ca. 250 und 1.000 Personen/Tag.

Das erfindungsgemäße Verfahren wurde wie folgt durchgeführt:

Zunächst wurde ein geschlossener Kreislauf zwischen dem Ablauf des Schwallwasserbeckens und dem Zulauf zum Schwimmbad aufgebaut. Dies erfolgt durch Vorsehen zusätzlicher Ein- und Ausgänge an bestimmten Stellen bei der Rohwasser- und Reinwasserverrohrung der Schwimmwasseraufbereüungsanlage.

Anschließend wird der Filter gemäß den für die Anlage erforderlichen Spüldaten gespült. Nach der Spülung wird die Umwälzanlage wieder auf Normalbetrieb gestellt. Nach einer ca. 10-minütigen Umwälzung des Schwimm- und Badebeckenwassers wird die Anlage abgestellt und darauf geachtet, dass kein Wasser aus der Prozessstrecke verlorengeht.

Es wird dann aus der zu reinigenden Prozessstrecke nur so viel Wasser entleert, wie andererseits durch die Einbringung der Desinfektionslösung wieder ergänzt wird. Anschließend wird eine wässerige Spüllösung zum Entkeimen und Reinigen in Form einer Tetrachlordecaoxid-Komplex-Dianion-Lösung in einer Konzentration, die einer Anfangskonzentration von Chlorit von etwa 0,1 bis 0,2 Mol/l entspricht, eingebracht.

Das Einbringen der Desinfektionslösung erfolgt, durch Eindosierung mittels Dosierpumpen während des Umwälzens in den zu reinigenden Teilen der Prozessstrecke der Badebeckenwasseraufbereitungsanlage.

Die Spüllösung wurde mittels einer speziellen frequenzgesteuerten Umwälzpumpe im sogenannten "Stop-and-Go"-Verfahren für definierte Zeitintervalle von jeweils etwa 1 bis 3 Std. umgewälzt und die Pumpe etwa 20 bis 40 Min. abgeschaltet, um eine definierte Eniwirkungszeit der Spüllösung zu gewährleisten.

In den sogenannten "Ruhephasen" ist es von Vorteil, wenn ein Spülluftgebläse vorhanden ist, um für 2 bis 5 Min. Spülluft in den Filter "einzublasen" und eine zusätzliche Auflockerung des Filtermaterials zu erreichen, welche den oxidativen Reinigungsvorgang der TCDO-Lösung im Filtermaterial begünstigt.

Nach etwa 3 Stunden Einsatz der Spüllösung sollte mittels TCDO-Reagenz überprüft werden, ob von der eingesetzten TCDO-Lösung noch Wirkstoff in der Spüllösung vorhanden ist Es sollte damit sichergestellt werden, dass genügend Wirksamkeit der Desinfektionslösung beim ganzen Reinigungsprozess vorhanden ist.

Danach erfolgt das Ablassen und Verwerfen der wässrigen Spüllösung. Dies wird nur bei den Rohrleitungen durchgeführt. Die restliche Spüllösung im Filter wird mittels Spülung des Filters über die Spülwasserleitung ausgetragen.

Anschließend wird der ursprüngliche Wasserlauf der Schwimm- und Badebeckenwasseraufbereitung wieder hergestellt.

Es sollte dann nach 24 bis 36 Std. Betriebszeit der Filter nochmals gespült werden.

Die Menge der Spüllösung betrug 1201 1 an Gebrauchslösung (Wirkstoff TCDO-Anion), welcher in insgesamt 9 m$^3$ Wasser eingebracht wurde und als Spüllösung eingesetzt wurde.

### Beispiel 3:

- Entkeimung und Reinigung des wasserführenden Systems mit einer erfindungsgemäßen Verfahrensvariante -

Es wurde wie in Beispiel 2 vorgegangen, wobei jedoch die Spüllösung definierte Zeitintervalle von jeweils etwa 1 bis 3 Std. umgewälzt und die Pumpe für etwa 30 bis 60 Min. abgeschaltet wurde, um eine definierte Einwirkung der Spüllösung zu gewährleisten.

Die entkeimende und reinigende Wirkung der Spüllösung wurde festgestellt durch die Desinfektionsmittelprüfung nach DIN EN 1276/August 1997 (Chemische Desinfektionsmittel und Antiseptika) sowie Untersuchungen und Versuche - labortechnisch und im großtechnischen Bereich - zur chemischen Reinigung und Desinfektion mikrobiologisch kontaminierter Aktivkornkohle aus verschiedenen öffentlichen Schwimmbadanlagen mit dem Tetrachlordecaoxid-Dianion (TCDO-Komplex).

Bei der praktischen Anwendung kann bzw. wird ein qualitativer Nachweis des TCDO-Anions in der Spüllösung durch das "HydroQuant" TCDO-Reagenz ermöglicht. Das "HydroQuant" TCDO-Reagenz ist im wesentlichen aus einer wässerigen Lösung von Eisen(III)chlorid mit der Summenformel $FeCl_3$ aufgebaut, stellt ein einfach zu handhabendes chemisches Prüfmittel dar und ermöglicht den Nachweis des TCDO-Anions in Form eines Farbumschlages (Färbung von hellgelb auf braun). Ist in der Spüllösung noch TCDO-Anion vorhanden, kommt es zu einer Braunfärbung, welche unabhängig vom pH-Wert der Lösung im weiten pH-Intervall von pH 3 bis pH 11 auftritt. Die Braunfärbung tritt auch unabhängig vom Gehalt an frei wirksamem Chlor auf. Auch Chlorkonzentrationen bis 100 mg/l frei wirksamen Chlors haben keinen Einfluss auf die Messbarkeit des TCDO-Anions in der Spüllösung. Die entkeimende und reinigende Wirkung der Spüllösung wurde demnach durch den Verbrauch des TCDO-Komplexes festgestellt.

### Beispiel 4:

- Entkeimung und Reinigung des wasserführenden Systems mit einer weiteren erfindungsgemäßen Verfahrensvariante -

Es wurde wie in Beispiel 2 ein geschlossener Kreislauf aufgebaut, jedoch mit genau festgelegten Abgrenzungen, wodurch nur ein ganz bestimmter Abschnitt der Prozessstrecke entkeimt und gereinigt werden sollte.

Dann wurde als hochkonzentrierte wässerige Spüllösung zum Entkeimen und Reinigen eine Tetrachlordecaoxid-Komplex-Dianion-Lösung in einer Konzentration, die einer Anfangskonzentration an Chlorit von etwa 1,5 Mol/l entspricht, eingebracht. Die Menge der Spüllösung betrug 1/100 bis 1/20 des Rohrvolumens. Die Spüllösung wurde mit Druckluft durch den abgetrennten Abschnitt befördert.

Die entkeimende und reinigende Wirkung wurde wie vorher in Beispiel 3 beschrieben bestimmt.

**Patentansprüche**

1. Verfahren zum Entkeimen und Reinigen von wasserführenden Systemen und Flächen, wobei das Entkeimen und Reinigen mittels Zugabe einer vorbestimmten Menge eines desinfizierenden Wirkstoffs in Form einer chloroxid-, halogen- und/oder peroxidhaltigen wässrigen Spüllösung erfolgt, **gekennzeichnet durch** die Verfahrensschritte:

   (1) Aufbauen eines geschlossenen Kreislaufs zwischen einem ersten und einem zweiten Punkt des wasserführenden Systems bzw. der wasserführenden Fläche, wobei die zu entkeimenden und zu reinigenden Systemkomponenten zwischen dem ersten und dem zweiten Punkt in fluidmechanischer Verbindung angeordnet sind und wobei diese Systemkomponenten wenigstens Rohre und Filter mit den Filtermaterialien, einschließlich Filterkohlen mit und ohne adsorptive Eigenschaften, umfassen,
   (2) Zugeben der wässrigen Spüllösung zum Entkeimen und Reinigen in den geschlossenen Kreislauf,
   (3) Entkeimen und Reinigen der Systemkomponenten unter periodischem Umwälzen der Spüllösung im geschlossenen Kreislauf in definierten Zeitintervallen,
   (4) Ablassen der wässrigen Spüllösung aus dem geschlossenen Kreislauf und Verwerfen derselben,
   (5) Wiederherstellen des ursprünglichen Wasserlaufs.

2. Verfahren nach Anspruch 1, **dadurch gekennzeichnet, dass** das wasserführende System eine Schwimm- oder Badebeckenanlage ist.

3. Verfahren nach Anspruch 2, **dadurch gekennzeichnet, dass** die Schwimm- oder Badebeckenanlage ein Schwallwasserbecken mit einem Ablauf und ein Schwimm- oder Badebecken mit einem Zulauf umfasst, wobei der Ablauf und der Zulauf eine fluidmechanische Verbindung aufweisen und wobei der erste Punkt in der fluidmechanischen Verbindung nahe dem Ablauf und der zweite Punkt in der fluidmechanischen Verbindung unmittelbar vor dem Zulauf vorgesehen ist und wobei das Aufbauen des geschlossenen Kreislaufs zwischen dem ersten und zweiten Punkt erfolgt.

4. Verfahren nach Anspruch 1, **dadurch gekennzeichnet, dass** das wasserführende System ein Warmsprudelbecken, insbesondere ein Hot-Whirl-Pool oder eine Whirl-Wanne, ist.

5. Verfahren nach Anspruch 1, **dadurch gekennzeichnet, dass** das wasserführende System ein Trinkwasserzuführungssystem in einer Hausinstallation, insbesondere ein Duschsystem, ist.

6. Verfahren nach Anspruch 5, **dadurch gekennzeichnet, dass** der Aufbau eines geschlossenen Kreislaufs in Schritt (1) zwischen dem Warmwasserboiler und dem Duschkopf erfolgt.

7. Verfahren nach Anspruch 1, **dadurch gekennzeichnet, dass** das wasserführende System ein Prozesswasseraufbereitungs-System im industriellen Bereich ist.

8. Verfahren nach einem der vorhergehenden Ansprüche, **dadurch gekennzeichnet, dass** das Umwälzen gemäß Schritt (3) in einer Vorzugsrichtung oder abwechselnd in eine der beiden Richtungen durchgeführt wird.

9. Verfahren nach mindestens einem der vorhergehenden Ansprüche, **dadurch gekennzeichnet, dass** die Spüllösung während weniger Minuten bis zu mehreren Stunden, insbesondere etwa 3 bis 12 h umgewälzt wird, wobei das Umwälzen ein oder mehrere Male unterbrochen werden kann.

10. Verfahren zum Entkeimen und Reinigen von wasserführenden Systemen und Flächen, insbesondere in Aufbereitungsanlagen, Rohrsystemen sowie Schwimm- und Badebeckenanlagen, wobei das Entkeimen und Reinigen mittels Zugabe einer hochkonzentrierten Spüllösung, enthaltend zumindest einen desinfizierenden Wirkstoff erfolgt, **gekennzeichnet durch** die Verfahrensschritte:

(a) Abtrennen eines definierten Abschnitts zwischen einem ersten und einem zweiten Punkt innerhalb des wasserführenden Systems, wobei die zu entkeimenden und zu reinigenden Systemkomponenten zwischen dem ersten und dem zweiten Punkt in fluidmechanischer Verbindung angeordnet sind,

(b) Einbringen der hochkonzentrierten Spüllösung in den abgetrennten Abschnitt,

(c) Durchspülen des abgetrennten Abschnitts mit der Spüllösung, gegebenenfalls unter Umwälzen,

(d) Entnahme einer Probe der Spüllösung aus dem abgetrennten Abschnitt,

(e) Überprüfen des Vorhandenseins des Wirkstoffs und/oder der Konzentration des Wirkstoffs und/oder der Entkeimungs- und Reinigungswirkung in der Spüllösungsprobe,

(f) gegebenenfalls Wiederholen der Schritte (c) bis (e),

(g) Ablassen der wässerigen Spüllösung aus dem abgetrennten Abschnitt und Verwerfen derselben,

(h) Wiederherstellen des ursprünglichen Wasserlaufs.

11. Verfahren nach Anspruch 10, **dadurch gekennzeichnet, dass** die Spüllösung in Schritt (c) mittels Gas unter erhöhtem Druck durchgespült wird.

12. Verfahren nach Anspruch 10, **dadurch gekennzeichnet, dass** die Spüllösung in Schritt (c) auf die Rohrinnenwände, Filter und/oder Filtermaterialien gesprüht wird.

13. Verfahren nach einem der vorhergehenden Ansprüche 1 bis 9 oder 10 bis 12, **dadurch gekennzeichnet, dass** von dem Entkeimen und Reinigen nach Schritt (3) oder nach Schritt (c), auch die im System vorhandenen Probeentnahmeleitungen und -entnahmehähne erfasst werden.

14. Verfahren nach einem der vorhergehenden Ansprüche, **dadurch gekennzeichnet, dass** als desinfizierender Wirkstoff eine chloroxid-, halogen- und/oder peroxidhaltige wässerige Spüllösung in einer Konzentration, die einer Anfangskonzentration an Chlorit von mindestens etwa 0,1 Mol/l, insbesondere von mindestens etwa 0,15 Mol/l, entspricht, eingesetzt wird.

15. Verfahren nach Anspruch 14, **dadurch gekennzeichnet, dass** in der chloroxidhaltigen wässerigen Lösung das Tetrachlordecaoxid-Komplex-Dianion $[Cl_4O_{10}]^{2-}$ vorliegt.

16. Verfahren nach mindestens einem der Ansprüche 14 oder 15, **dadurch gekennzeichnet, dass** die wässerige Lösung des Tetrachlordecaoxid-Komplex-Dianions erhalten wird, indem eine einen pH-Wert $\leq 3$ aufweisende sulfationenhaltige, wässerige Lösung mit einer darin stabilen Peroxoverbindung versetzt, und diese Lösung anschließend mit der alkalischen, wässerigen Lösung eines Chlorits vermischt wird.

17. Verfahren nach Anspruch 16, **dadurch gekennzeichnet, dass** die sulfationenhaltige, wässerige Lösung mit einer solchen Menge einer darin stabilen Peroxoverbindung versetzt wird, dass sich eine Konzentration an Peroxoverbindung in der Lösung von etwa 0,001 bis 0,01 molar ergibt.

18. Verfahren nach Anspruch 16 oder 17, **dadurch gekennzeichnet, dass** die sulfationenhaltige, wässerige Lösung mit einer darin stabilen Peroxoverbindung versetzt und diese Lösung anschließend mit der alkalischen, wässerigen Lösung eines Chlorits in einer Menge vermischt wird, so dass sich ein pH-Wert von über 7,0, insbesondere zwischen 7,5 und 8,0, einstellt.

19. Verfahren nach mindestens einem der Ansprüche 16 bis 18, **dadurch gekennzeichnet, dass** als Peroxoverbindung eine anorganische Peroxoverbindung in Form von Wasserstoffperoxid, einem Persulfat, Percarbonat, Perborat oder einem Peroxid eines Alkali- oder Erdalkalimetalls verwendet wird.

20. Verfahren nach mindestens einem der Ansprüche 16 bis 19, **dadurch gekennzeichnet, dass** als Chlorit ein Alkali- und/oder Erdalkalichlorit eingesetzt wird.

21. Verfahren nach mindestens einem der Ansprüche 16 bis 20, **dadurch gekennzeichnet, dass** die sulfationenhaltige, wässerige Lösung einen pH-Wert $\leq 1$ aufweist.

22. Verfahren nach mindestens einem der Ansprüche 16 bis 21, **dadurch gekennzeichnet, dass** zur Herstellung der sulfationenhaltigen, wässerigen Lösung demineralisiertes Wasser verwendet wird.

23. Verfahren nach mindestens einem der Ansprüche 16 bis 22, **dadurch gekennzeichnet, dass** die Spüllösung ein wasserlösliches Phosphat enthält.

**24.** Verfahren nach mindestens einem der vorhergehenden Ansprüche, **dadurch gekennzeichnet, dass** eine ständige Zudosierung von Desinfektionsmitteln vorgesehen ist.

**25.** Vorrichtung zum Entkeimen und Reinigen von wasserführenden Systemen und Flächen, wobei die wasserführenden Systeme und Flächen wenigstens Rohre und einen Filter umfassen, und wobei die Vorrichtung Mittel zum Einbringen einer vorbestimmten Menge zumindest eines desinfizierenden Wirkstoffs aufweist, **gekennzeichnet durch**:

(1) Mittel zum Erzeugen eines geschlossenen Kreislaufs zwischen einem ersten und einem zweiten Punkt des wasserführenden Systems bzw. der wasserführenden Fläche, wobei die zu entkeimenden und zu reinigenden Systemkomponenten zwischen dem ersten und zweiten Punkt in fluidmechanischer Verbindung angeordnet sind und wenigstens die Rohre und den Filter umfassen,
(2) die Mittel zum Einbringen, wobei diese zum Einbringen der vorbestimmten Menge des zumindest einen desinfizierenden Wirkstoffs in den geschlossenen Kreislauf vorgesehen sind, und wobei der desinfizierende Wirkstoff eine chloroxid-, halogen- und/oder peroxidhaltige wässrige Spüllösung zum Entkeimen und Reinigen ist,
(3) wenigstens eine Pumpe zum Umwälzen der Spüllösung in dem geschlossenen Kreislauf zum Entkeimen und Reinigen der Systemkomponenten,
(4) wenigstens einen Ablass für die wässrige Spüllösung aus dem geschlossenen Kreislauf und
(5) Mittel zur Wiederherstellung des ursprünglichen Wasserlaufs.

**26.** Vorrichtung nach Anspruch 25, **dadurch gekennzeichnet, dass** das wasserführende System eine Schwimm- oder Badebeckenanlage ist.

**27.** Vorrichtung nach Anspruch 26, **dadurch gekennzeichnet, dass** die Schwimm- oder Badebeckenanlage ein Schwallwasserbecken mit einem Ablauf und ein Schwimm- oder Badebecken mit einem Zulauf umfasst, wobei der Ablauf und der Zulauf eine fluidmechanische Verbindung aufweisen und wobei der erste Punkt in der fluidmechanischen Verbindung nahe dem Ablauf und der zweite Punkt in der fluidmechanischen Verbindung unmittelbar vor dem Zulauf vorgesehen sind.]

**28.** Vorrichtung zum Entkeimen und Reinigen von wasserführenden Systemen und Flächen, insbesondere in Aufbereitungsanlagen, Rohrsystemen sowie Schwimm- und Badebeckenanlagen, umfassend, Mittel zum Einbringen einer hochkonzentrierten Spüllösung, enthaltend zumindest einen desinfizierenden Wirkstoff, und Mittel zum Überprüfen des Vorhandenseins des Wirkstoffs und/oder der Konzentration des Wirkstoffs und/oder der Entkeimungs- und Reinigungswirkung, **gekennzeichnet durch**:

(a) Mittel zum Abtrennen eines definierten Abschnitts zwischen einem ersten und einem zweiten Punkt innerhalb des wasserführenden Systems, wobei die zu entkeimenden und zu reinigenden Systemkomponenten zwischen dem ersten und dem zweiten Punkt in fluidmechanischer Verbindung angeordnet sind,
(b) die Mittel zum Einbringen, wobei diese zum Einbringen der hochkonzentrierten Spüllösung in den abgetrennten Abschnitt vorgesehen sind,
(c) Mittel zum Durchspülen des abgetrennten Abschnitts mit der Spüllösung zum Entkeimen und Reinigen der Systemkomponenten,
(d) Mittel zur Entnahme einer Probe der Spüllösung aus dem abgetrennten Abschnitt,
(e) Mittel zum Ablassen der wässerigen Spüllösung aus dem abgetrennten Abschnitt und
(f) Mittel zum Wiederherstellen des ursprünglichen Wasserlaufs.

**Claims**

**1.** A method of disinfecting and purifying water-conveying systems and surfaces, wherein disinfection and purification are effected by adding a set quantity of a disinfectant in the form of a chlorine oxide or halogen and/or peroxide containing aqueous rinsing solution, **characterised by** the following steps:

(1) Constructing a closed circuit between a first and a second point of the water-conveying system for the water-conveying surface, wherein the components of the system for disinfecting and purification are disposed in a mechanical fluid connection between the first and the second point and wherein the said components comprise at least pipes, filters and filter materials, including filtering charcoal with or without adsorptive properties,
(2) Adding an aqueous rinsing solution for disinfecting and purifying to the closed circuit,
(3) Disinfecting and purifying the system components by periodically circulating the rinsing solution in the closed circuit at defined intervals,
(4) Discharging the aqueous rinsing solution from the closed circuit and dumping the solution, and
(5) Reconnecting the original water channel.

2. A method according to claim 1, **characterised in that** the water-conveying system is a swimming or bathing-pool establishment.

3. A method according to claim 2, **characterised in that** the swimming or bathing-pool establishment comprises a wave pool with an outlet and a swimming or bathing pool with an inlet, wherein the inlet and outlet have a mechanical fluid connection and wherein the first point in the connection is near the outlet and the second point in the connection is immediately in front of the inlet and wherein the closed circuit is constructed between the first and the second point.

4. A method according to claim 1, **characterised in that** the water-conveying system is a water whirlpool, especially a hot whirlpool or a whirl bathtub.

5. A method according to claim 1, **characterised in that** the water-conveying system is a drinking-water supply system in a domestic installation, especially a shower system.

6. A method according to claim 5, **characterised in that** the closed circuit in step (1) is constructed between the water boiler and the shower head.

7. A method according to claim 1, **characterised in that** the water-conveying system is an industrial system for providing process water.

8. A method according to any of the preceding claims, **characterised in that** circulation in step (3) is effected in a preferred direction or in one of the two directions alternately.

9. A method according to at least one of the preceding claims, **characterised in that** the rinsing solution is circulated for a few minutes to a number of hours, especially about 3 to 12 hours, wherein circulation can be interrupted one or more times.

10. A method of disinfecting and purifying water-conveying systems and surfaces, especially in processing installations, pipe systems and/or swimming or bathing-pool establishments, wherein disinfection and purification are effected by adding a highly concentrated rinsing solution containing at least one disinfectant, **characterised by** the following steps:

(a) Dividing off a defined portion between a first and a second point in the water-conveying system, wherein the components of the system for disinfection and purifying are disposed between the first and the second point in a mechanical fluid connection,
(b) Introducing the highly-concentrated washing solution into the divided-off portion,
(c) Thoroughly rinsing the divided-off portion with the rinsing solution, circulated if required,
(d) Removing a sample of the rinsing solution from the divided-off portion,
(e) Checking the presence of the disinfectant and/or the concentration of the disinfectant and/or the disinfecting and/or purifying effect in the sample of the rinsing solution,
(f) Repeating steps (c) to (e) if required,
(g) Discharging the aqueous rinsing solution from the divided-off portion and dumping the solution, and
(h) Reconnecting the original water channel.

11. A method according to claim 10, **characterised in that** the rinsing solution in step (c) is scavenged by gas at elevated pressure.

12. A method according to claim 10, **characterised in that** the rinsing solution in step (c) is sprayed on to the inner surfaces of the pipes, the filters and/or the fitter materials.

13. A method according to any of the preceding claims 1 to 9 or 10 to 12, **characterised in that** disinfection and purification in step (3) or step (c) also applies to the pipes and taps in the system for withdrawing samples.

**14.** A method according to any of the preceding claims, **characterised in that** the disinfectant is a chlorine oxide or halogen and/or peroxide containing aqueous rinsing solution in a concentration corresponding to an initial chlorite concentration of at least about 0.1 mol/l, especially at least about 0.15 mol/l.

**15.** A method according to claim 14, **characterised in that** the chlorine oxide containing aqueous solution contains the tetrachloride decaoxide complex dianion $[Cl_4O_{10}]^{2-}$.

**16.** A method according to at least one of claim 14 or claim 15, **characterised in that** the aqueous solution of the tetrachloride decaoxide complex dianion is obtained by mixing an aqueous solution containing sulphate ions and having a pH $\leq$ 3 with a peroxo compound stable therein and mixing the resulting solution with an alkaline aqueous solution of the chlorite.

**17.** A method according to claim 16, **characterised in that** the aqueous solution containing sulphate ions is mixed with a quantity of a peroxo compound stable therein such that the concentration of the peroxo compound in the solution is about 0.001 to 0.01 mol.

**18.** A method according to claim 16 or claim 17, **characterised in that** the aqueous solution containing sulphate ions is mixed with a peroxo compound stable therein and the resulting solution is mixed with the alkaline aqueous solution of a chlorite in a quantity such that the pH is over 7.0, especially between 7.5 and 8.0.

**19.** A method according to at least one of claims 16 to 18, **characterised in that** the peroxo compound is an inorganic peroxo compound in the form of hydrogen peroxide or a persulphate, percarbonate, perborate or a peroxide of an alkali metal or alkaline earth metal.

**20.** A method according to at least one of claims 16 to 18, **characterised in that** the chlorite is an alkali metal and/or alkaline earth chlorite.

**21.** A method according to at least one of claims 16 to 20, **characterised in that** the aqueous solution containing sulphate ions has a pH $\leq$ 1.

**22.** A method according to at least one of claims 16 to 21, **characterised in that** demineralised water is used for producing the aqueous solution containing sulphate ions.

**23.** A method according to at least one of claims 16 to 22, **characterised in that** the rinsing solution contains a water-soluble phosphate.

**24.** A method according to at least one of the preceding claims, **characterised by** continuous addition of disinfectants.

**25.** A method of disinfecting and purifying water-conveying systems and surfaces, wherein the water-conveying systems and surfaces at least comprise pipes and a filter and wherein the device comprises means for introducing a set amount of at least one disinfectant, **characterised by**

(1) Means for producing a closed circuit between a first and a second point on the water-conveying system for the water-conveying surface, wherein the components of the system for disinfecting and purifying are disposed between the first and the second point in a mechanical fluid connection and comprise at least the pipes and the filters.
(2) The introducing means, wherein the said means are provided for introducing the set quantity of the at least one disinfectant into the closed circuit and wherein the disinfectant is a chlorine oxide or halogen and/or peroxide-containing aqueous rinsing solution used for disinfecting and purifying.
(3) At least one pump for circulating the rinsing solution in the closed circuit for disinfecting and purifying the system components.
(4) At least one outlet for the aqueous rinsing solution from of the closed circuit and
(5) Means for reconnecting the original water channel.

**26.** A device according to claim 25, **characterised in that** the water-containing system is a swimming or bathing-pool establishment.

**27.** A device according to claim 26, **characterised in that** the swimming or bathing-pool establishment comprises a wave pool with an outlet and a swimming or bathing pool with an inlet, wherein the outlet and the inlet have a mechanical fluid connection and wherein the first point in the mechanical fluid connection is near the outlet and the second point in the mechanical fluid connection is immediately in front of the inlet.

**28.** A device for disinfecting and purifying water-conveying systems and surfaces, especially in processing installations, pipe systems or swimming and bathing-pool establishments, comprising means for introducing a highly concentrated rinsing solution containing at least one disinfectant and means for checking the presence of the disinfectant and/or the concentration thereof and/or the disinfecting and purifying effect, **characterised by**:

(a) Means for dividing off a defined portion between a first and a second point in the water-conveying system, wherein the system components for disinfecting and cleaning and purifying are disposed between the first and the second point in a mechanical fluid connection,
(b) The introducing means, wherein the said means are for introducing the highly concentrated rinsing solution into the divided-off portion,
(c) Means for thoroughly rinsing the divided-off portion with the rinsing solution in order to disinfect and purify the system components,
(d) Means for withdrawing a sample of the washing solution from the divided-off portion,
(e) Means for discharging the aqueous rinsing solution from the divided-off portion and
(f) Means for reconnecting the original water channel.

**Revendications**

**1.** Procédé pour la stérilisation et le nettoyage de systèmes et de surfaces véhiculant de l'eau, la stérilisation et le nettoyage s'effectuant avec l'addition d'une quantité prédéfinie d'un agent désinfectant sous la forme d'une solution de lavage aqueuse à base d'oxyde de chlore, d'halogène et/ou de peroxyde, **caractérisé par** les étapes de procédé suivantes :

(1) mise en place d'un circuit fermé entre un premier et un second point du système véhiculant de l'eau ou de la surface véhiculant de l'eau, les composants du système à stériliser et à nettoyer étant disposés entre le premier et le second point en liaison fluidique et mécanique, et ces composants du système comportant au moins des tuyaux et des filtres avec les matériaux de filtre, y compris des charbons à filtre avec et sans propriétés adsorbantes,
(2) addition de la solution de lavage aqueuse pour la stérilisation et le nettoyage dans le circuit fermé,
(3) stérilisation et nettoyage des composants du système avec circulation périodique de la solution de lavage en circuit fermé dans des intervalles de temps définis,
(4) évacuation de la solution de lavage aqueuse du circuit fermé et rejet de cette solution,
(5) rétablissement de la circulation d'eau initiale.

**2.** Procédé selon la revendication 1, **caractérisé en ce que** le système véhiculant de l'eau est une installation de piscine ou une installation de bassin de bains.

**3.** Procédé selon la revendication 2, **caractérisé en ce que** l'installation de piscine ou de bassin de bain comporte un bassin à projections d'eau avec un écoulement et un bassin de piscine ou un bassin de bain avec une arrivée, l'écoulement et l'arrivée présentant une liaison mécanique et fluidique, et le premier point étant prévu dans la liaison fluidique et mécanique à proximité de l'écoulement et le second point dans la liaison fluidique et mécanique à proximité de l'arrivée, et l'établissement du circuit fermé s'effectuant entre le premier et le second point.

**4.** Procédé selon la revendication 1, **caractérisé en ce que** le système véhiculant de l'eau est un bassin à eau chaude bouillonnante, en particulier un bassin à remous d'eau chaude ("hot-whirl-pool") ou une baignoire à remous.

**5.** Procédé selon la revendication 1, **caractérisé en ce que** le système véhiculant de l'eau est un système d'alimentation en eau potable dans une installation domestique, en particulier un système de douche.

**6.** Procédé selon la revendication 5, **caractérisé en ce que** la mise en place d'un circuit fermé à l'étape (1) s'effectue entre le chauffe-eau à accumulation et la pomme de douche.

**7.** Procédé selon la revendication 1, **caractérisé en ce que** le système véhiculant de l'eau est un système de préparation d'eau de processus dans le secteur industriel.

**8.** Procédé selon l'une des revendications précédentes, **caractérisé en ce que** la circulation selon l'étape (3) est effectuée dans un sens préférentiel ou alternativement dans l'une des deux directions.

**9.** Procédé selon au moins l'une des revendications précédentes, **caractérisé en ce que** la solution de lavage est mise en circulation au moins pendant quelques minutes jusqu'à plusieurs heures, en particulier environ 3 à 12 heures, la circulation pouvant être interrompue une ou plusieurs fois.

**10.** Procédé pour la stérilisation et le nettoyage de systèmes et de surfaces véhiculant de l'eau, en particulier dans des installations de traitement, des systèmes tubulaires ainsi que des installations de piscine et de bassins de bain, la stérilisation et le nettoyage s'effectuant avec l'addition d'une solution de lavage hautement concentrée, contenant au moins un agent désinfectant, **caractérisé par** les étapes de procédé suivantes :

(a) séparation d'une partie définie entre un premier et un second point à l'intérieur du système véhiculant de l'eau, les composants du système à stériliser et à nettoyer étant disposés en liaison fluidique et mécanique entre le premier et le second point,
(b) introduction de la solution de lavage hautement concentrée dans la partie séparée,
(c) lavage de la partie séparée avec la solution de lavage, éventuellement avec circulation,
(d) prélèvement d'un échantillon de la solution de lavage de la partie séparée,
(e) contrôle de la présence de l'agent actif et/ou de la concentration de l'agent actif et/ou de l'effet de stérilisation et de nettoyage dans l'échantillon de solution de lavage,
(f) éventuellement répétition des étapes (c) à (e),
(g) évacuation de la solution de lavage aqueuse de la partie séparée et rejet de cette solution,
(h) rétablissement de la circulation d'eau initiale.

**11.** Procédé selon la revendication 10, **caractérisé en ce que** la solution de lavage est lavée à l'étape (c) avec du gaz sous pression élevée.

**12.** Procédé selon la revendication 10, **caractérisé en ce que** la solution de lavage est vaporisée à l'étape (c) sur les parois intérieures des tuyaux, sur les filtres et/ou matériaux de filtre.

**13.** Procédé selon l'une des revendications précédentes 1 à 9 ou 10 à 12, **caractérisé en ce qu'**également les conduites et robinets de prélèvement d'échantillon présents dans le système sont pris en compte par la stérilisation et le nettoyage après l'étape (3) ou l'étape (c).

**14.** Procédé selon l'une des revendications précédentes, **caractérisé en ce que**, comme agent désinfectant, on utilise une solution de lavage aqueuse à base d'oxyde de chlore, d'halogène et/ou de peroxyde dans une concentration qui correspond à une concentration initiale de chlorite d'au moins d'environ 0,1 mol/l, en particulier d'au moins environ 0,15 mol/l.

**15.** Procédé selon la revendication 14, **caractérisé en ce que** le dianion de complexe de décaoxyde de tétrachlorure $[Cl_4O_{10}]^{2-}$ est présent dans la solution aqueuse à base d'oxyde de chlore.

**16.** Procédé selon au moins l'une des revendications 14 ou 15, **caractérisé en ce que** la solution aqueuse du dianion de complexe de décaoxyde de tétrachlorure est obtenue en mélangeant une solution aqueuse, à base d'ions de sulfate et présentant un pH $\leq$ 3, avec un composé peroxo stable à l'intérieur et cette solution est mélangée ensuite avec la solution aqueuse et alcaline d'un chlorite.

**17.** Procédé selon la revendication 16, **caractérisé en ce que** la solution aqueuse, à base d'ions de sulfate, est mélangée avec une telle quantité d'un composé peroxo stable à l'intérieur pour qu'on obtienne une concentration de composé peroxo dans la solution d'environ 0,001 jusqu'à 0,01 molaire.

**18.** Procédé selon la revendication 16 ou 17, **caractérisé en ce que** la solution aqueuse, à base d'ions de sulfate, est mélangée avec un composé peroxo stable à l'intérieur et cette solution est mélangée ensuite avec la solution aqueuse et alcaline d'un chlorite dans une quantité telle qu'on obtient un pH supérieur à 7,0, en particulier compris entre 7,5 et 8,0.

**19.** Procédé selon l'une des revendications 16 à 18, **caractérisé en ce que**, comme composé peroxo, on utilise un composé peroxo inorganique sous la forme de peroxyde d'hydrogène, d'un persulfate, percarbonate, perborate ou d'un peroxyde ou d'un métal alcalin ou d'un métal alcalino-terreux.

**20.** Procédé selon au moins l'une des revendications 16 à 19, **caractérisé en ce que**, comme chlorite, on utilise un chlorite alcalin et/ou un chlorite alcalino-terreux.

**21.** Procédé selon au moins l'une des revendications 16 à 20, **caractérisé en ce que** la solution aqueuse, à base d'ions de sulfate, présente un pH ≤ 1.

**22.** Procédé selon au moins l'une des revendications 16 à 21, **caractérisé en ce qu'**on utilise de l'eau déminéralisée pour fabriquer la solution aqueuse à base d'ions de sulfate.

**23.** Procédé selon au moins l'une des revendications 16 à 22, **caractérisé en ce que** la solution de lavage contient un phosphate soluble dans l'eau.

**24.** Procédé selon au moins l'une des revendications précédentes, **caractérisé en ce qu'**une addition dosée constante de désinfectants est prévue.

**25.** Dispositif pour la stérilisation et le nettoyage de systèmes et de surfaces véhiculant de l'eau, les systèmes et surfaces véhiculant de l'eau comportant au moins des tuyaux et un filtre, et le dispositif présentant des moyens pour l'introduction d'une quantité prédéfinie d'au moins un agent désinfectant, **caractérisé par**

(1) des moyens pour générer un circuit fermé entre un premier et un second point du système véhiculant de l'eau ou de la surface véhiculant de l'eau, les composants du système à stériliser et à nettoyer étant disposés entre le premier et le second point en liaison fluidique et mécanique et comportant au moins les tuyaux et le filtre,
(2) des moyens pour l'introduction, ces moyens étant prévus pour l'introduction de la quantité prédéfinie de l'au moins un agent désinfectant dans le circuit fermé, et l'agent désinfectant étant une solution de lavage aqueuse contenant de l'oxyde de chlore, de l'halogène et/ou du peroxyde pour la stérilisation et le nettoyage,
(3) au moins une pompe pour la circulation de la solution de lavage dans le circuit fermé pour la stérilisation et le nettoyage des composants du système,
(4) au moins une évacuation pour la solution de lavage aqueuse sortant du circuit fermé et
(5) des moyens pour le rétablissement de la circulation d'eau initiale.

**26.** Dispositif selon la revendication 25, **caractérisé en ce que** le système véhiculant de l'eau est une installation de piscine ou une installation de bassin de bain.

**27.** Dispositif selon la revendication 26, **caractérisé en ce que** l'installation de piscine ou de bassin de bain comporte un bassin à projections d'eau avec une évacuation et un bassin de piscine ou un bassin de bain avec une arrivée, l'arrivée et l'évacuation présentant une liaison fluidique et mécanique, et le premier point et le second point étant prévus respectivement dans la liaison fluidique et mécanique à proximité de l'évacuation et dans la liaison fluidique et mécanique directement avant l'arrivée.

**28.** Dispositif pour la stérilisation et le nettoyage de systèmes et de surfaces véhiculant de l'eau, en particulier dans des installations de traitement, des systèmes de tuyaux et des installations de piscine et de bassin de bain, comprenant des moyens pour l'introduction d'une solution de lavage hautement concentrée, contenant au moins un agent désinfectant, et des moyens pour le contrôle de la présence de l'agent actif et/ou de la concentration de l'agent actif et/ou de l'effet de stérilisation et de nettoyage, **caractérisé par** :

(a) des moyens pour séparer une partie définie entre un premier et un second point à l'intérieur du système véhiculant de l'eau, les composants du système à stériliser et à nettoyer étant disposés entre le premier et le second point dans une liaison fluidique et mécanique,
(b) les moyens pour l'introduction, ceux-ci étant prévus pour l'introduction de la solution de lavage hautement concentrée dans la partie séparée,
(c) des moyens pour le lavage de la partie séparée avec la solution de lavage pour la stérilisation et le nettoyage des composants du système,
(d) des moyens pour le prélèvement d'un échantillon de la solution de lavage de la partie séparée,
(e) des moyens pour l'évacuation de la solution de lavage aqueuse de la partie séparée et

(f) des moyens pour le rétablissement de la circulation d'eau initiale.

Figur 1

| | |
|---|---|
| 1 | Absperrung Zulaufleitung |
| 2 | Wasserzähler |
| 3 | Absperrarmatur |
| 4 | Schmutzfilter |
| 5 | Rücklauf Zirkulationsleitung |
| 6 | Absperrarmatur |
| 7 | Entleerungsarmatur mit Entlüftung |
| 8 | Absperrarmatur |
| 9 | Dosierpumpe 1 |
| 10 | Dosierbehälter 1 |

| | |
|---|---|
| 11 | Dosierpumpe 2 |
| 12 | Dosierbehälter 2 |
| 13 | Stromanschluß Dosierpumpe 1 |
| 14 | Absperrarmatur |
| 15 | Stromanschluß Dosierpumpe 2 |
| 16 | Zirkulationspumpe (Reinigungskreislauf) |
| 17 | Absperrarmatur |
| 18 | Stromanschluß Zirkulationspumpe |
| 19 | Zulauf (Reinigungskreislauf) bzw. Versorgungsleitungen |
| 20 | Absperrarmatur |

Figur 2

Intensität der Verkeimung:

——— Stufe 1 (schwache Verkeimung)

▨▨▨ Stufe 2 (mittlere Verkeimung)

▬▬▬ Stufe 3 (starke Verkeimung)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3229219
- DE 3744355 C1
- DE 10010255 A1
- FR 2676218 A1